# EUROPEAN PATENT APPLICATION

(11) **EP 4 450 951 A1**
(43) Date of publication of application: **23.10.2024**
(21) Application number: 22907065.1
(22) Date of filing: 31.10.2022
(51) Int. Cl.: G01N 15/02, C12M 1/34, C12Q 1/06, G01N 15/06, G01N 21/27

(54) **MEASUREMENT DEVICE, MEASUREMENT METHOD, AND PROGRAM**

(30) Priority: 15.12.2021 JP 2021203356
(71) Applicant: Sony Group Corporation, Tokyo 108-0075 (JP)
(72) Inventor: TAKATSUKA, Susumu, Tokyo 108-0075 (JP); SATO, Hidehito, Tokyo 108-0075 (JP); TETSUKAWA, Hiroki, Tokyo 108-0075 (JP)
(74) Representative: MFG Patentanwälte Meyer-Wildhagen Meggle-Freund Gerhard PartG mbB
(86) International application number: PCT/JP2022/040816
(87) International publication number: WO 2023/112532

(57) **Abstract**

A measurement device includes a vision sensor that has a plurality of pixels, each outputting an event signal in an asynchronous manner according to a luminance change of an incident light, a cluster forming unit that forms, as a cluster, a point group of events generated by identical target particles on the basis of an event signal input in a predetermined period, a feature amount calculating unit that calculates the feature amount of the cluster, and a cluster classifying unit that classifies the cluster on the basis of the feature amount calculated by the feature amount calculating unit.

## Description

### [Technical Field]

The present technique relates to a measurement device, a measurement method, and a program, and relates particularly to a technique for measurement using a vision sensor.

### [Background Art]

A measurement device has been proposed which measures the biomass of phytoplankton by irradiating phytoplankton with excitation light at a predetermined wavelength to excite the phytoplankton and then measuring the intensity of fluorescence emitted from the excited phytoplankton (see PTL 1, for example).

### [Citation List]

### [Patent Literature]

[PTL 1]
JP 2019-165687A

### [Summary]

### [Technical Problem]

Unfortunately, the above-described measurement device can only measure phytoplankton excited by excitation light. However, it is assumed that measurements are conducted on zooplankton, organisms such as larvae of underwater life, and non-living objects such as microplastics, dust, sand, marine snow, and air bubbles as well as phytoplankton in, for example, oceanographic surveys, and thus accurate classification of such objects has been demanded.

Accordingly, an object of the present technique is to classify target particles with high accuracy.

### [Solution to Problem]

A measurement device according to the present technique includes a vision sensor that has a plurality of pixels, each outputting an event signal in an asynchronous manner according to a luminance change of an incident light, a cluster forming unit that forms, as a cluster, a point group of events generated by identical target particles on the basis of the event signal input in a predetermined period, a feature amount calculating unit that calculates the feature amount of the cluster, and a cluster classifying unit that classifies the cluster on the basis of the feature amount calculated by the feature amount calculating unit.

Thus, the measurement device can classify the cluster on the basis of the event signal input from the vision sensor.

### [Brief Description of Drawings]

[Fig. 1]

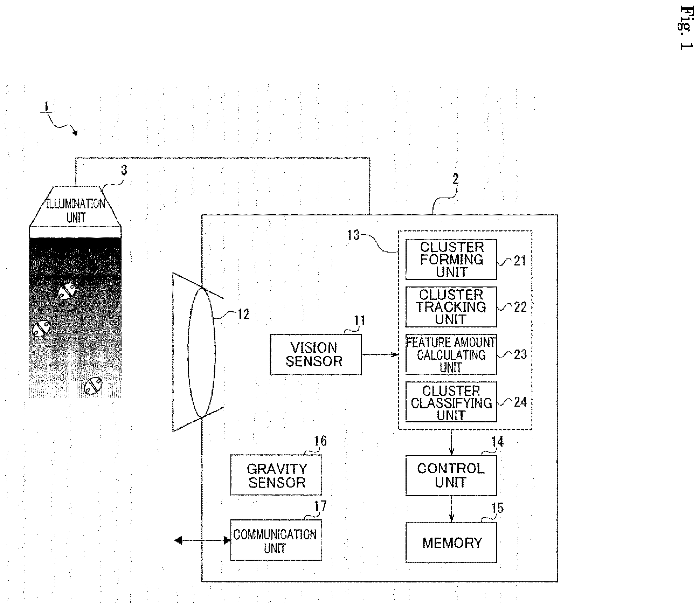

   Fig. 1 is an explanatory drawing of the configuration of a measurement device.
[Fig. 2]

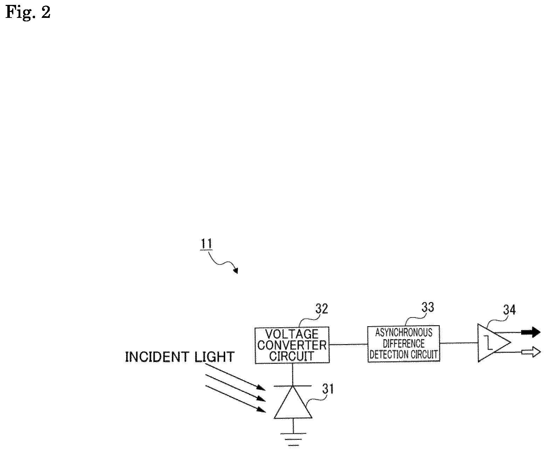

   Fig. 2 is an explanatory drawing of the configuration of a vision sensor.
[Fig. 3]

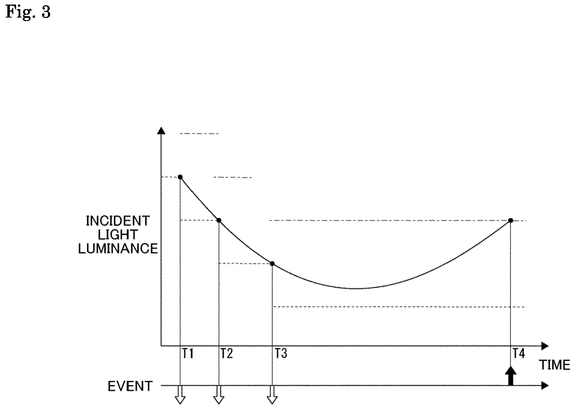

   Fig. 3 is an explanatory drawing of the outline of an event signal output from the vision sensor.
[Fig. 4]

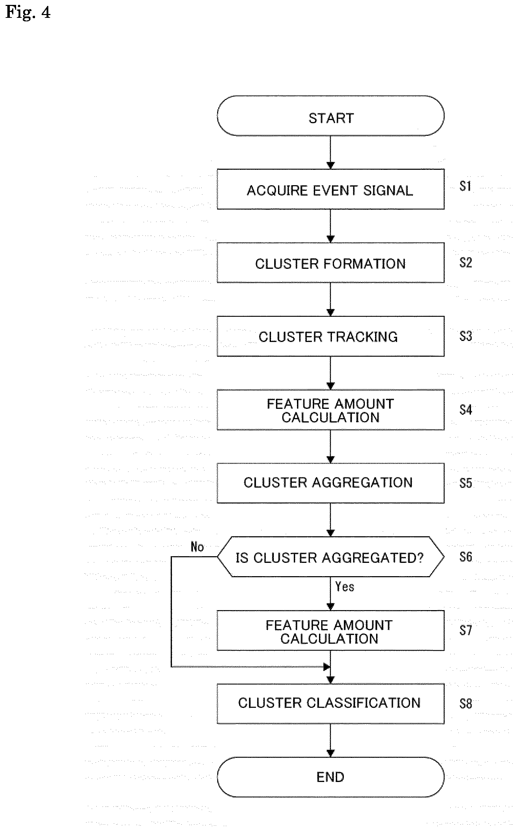

   Fig. 4 is a flowchart for describing the flow of signal analysis.
[Fig. 5]

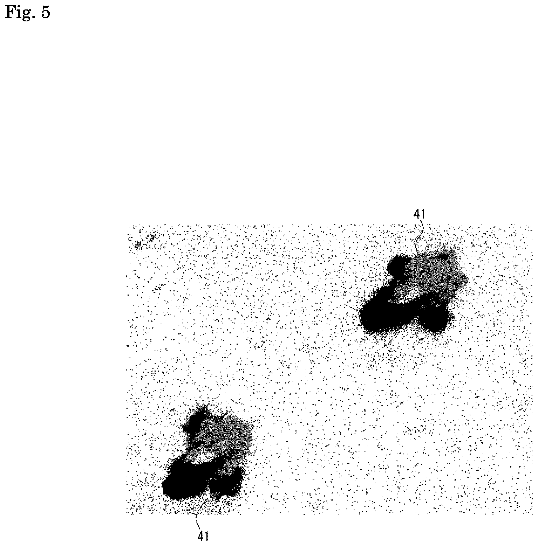

   Fig. 5 is an explanatory drawing of a captured image based on the event signal.
[Fig. 6]

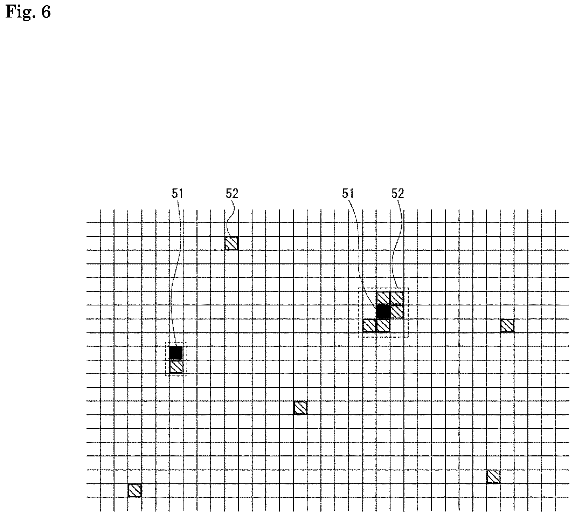

   Fig. 6 is an explanatory drawing of an event in a captured image.
[Fig. 7]

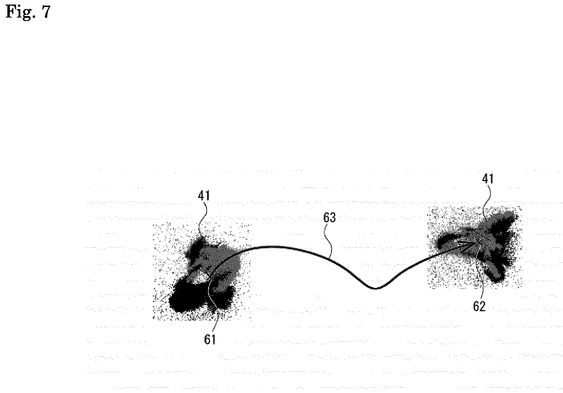

   Fig. 7 is an explanatory drawing showing calculation of a mean velocity of a cluster.
[Fig. 8]

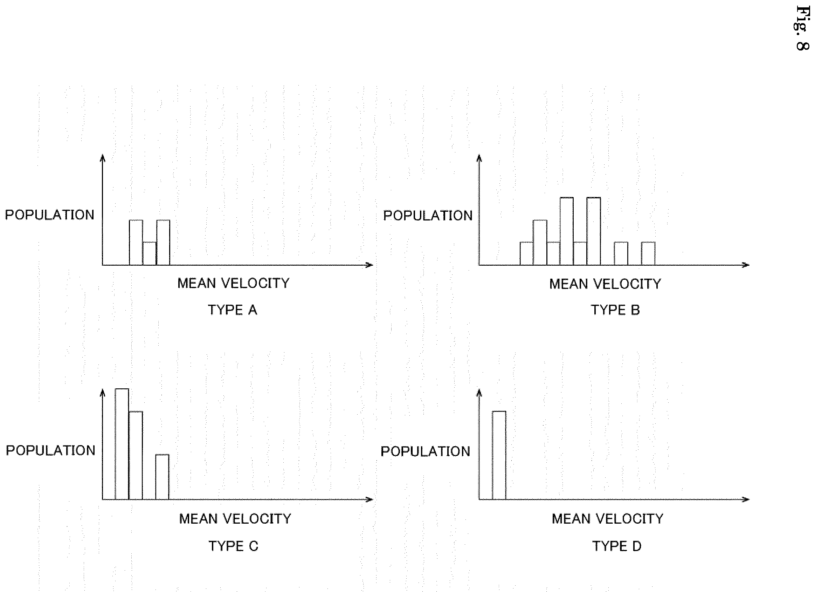

   Fig. 8 shows a mean velocity for each type.
[Fig. 9]

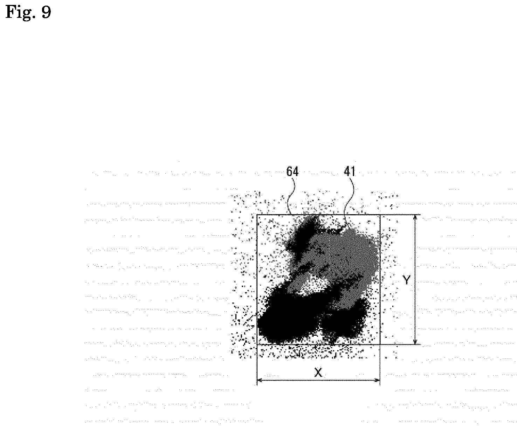

   Fig. 9 is an explanatory drawing showing calculation of a body length of the cluster.
[Fig. 10]

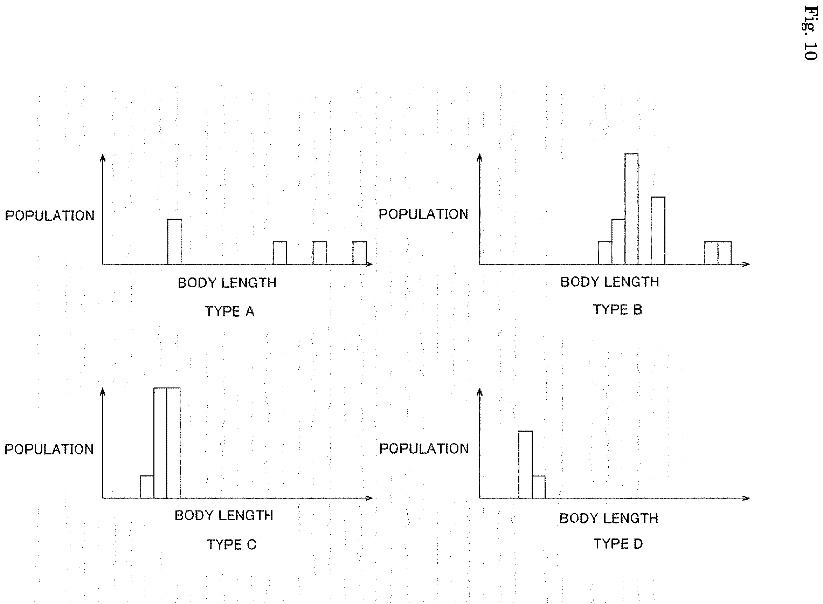

   Fig. 10 is an explanatory drawing of a body length for each type.
[Fig. 11]

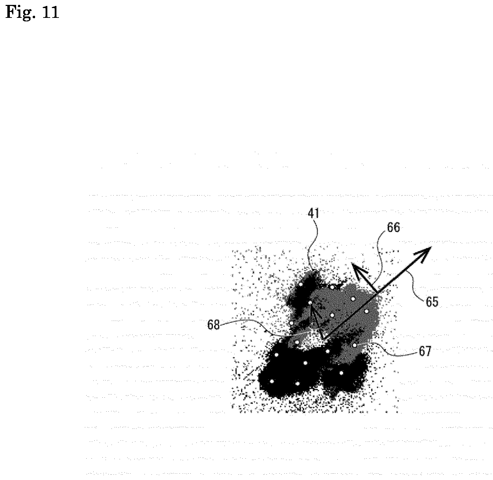

   Fig. 11 is an explanatory drawing showing calculation of a body width of the cluster.
[Fig. 12]

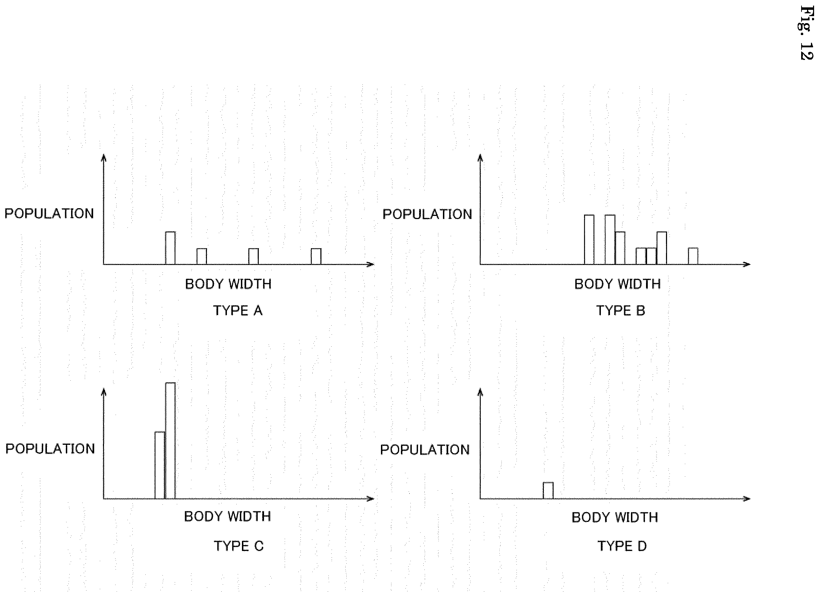

   Fig. 12 is an explanatory drawing of a body width for each type.
[Fig. 13]

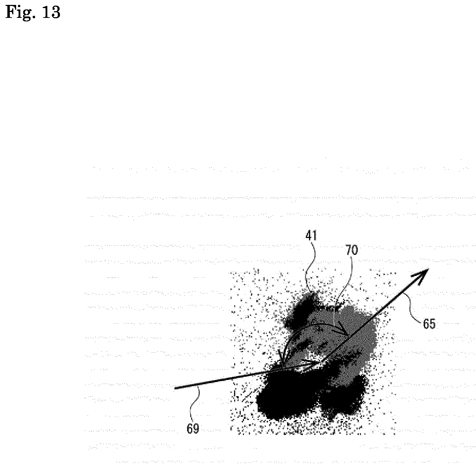

   Fig. 13 is an explanatory drawing showing calculation of a frequency of an angular change.
[Fig. 14]

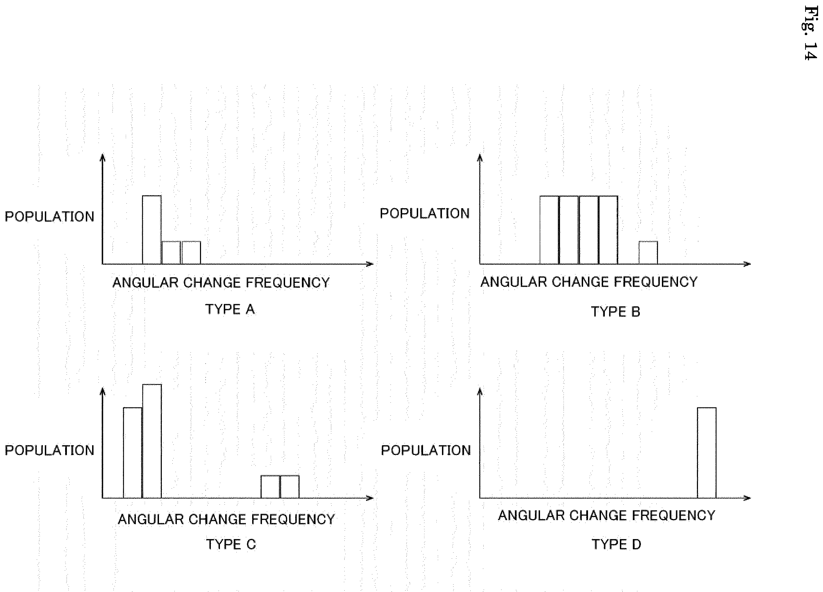

   Fig. 14 is an explanatory drawing showing a frequency of an angular change for each type.
[Fig. 15]

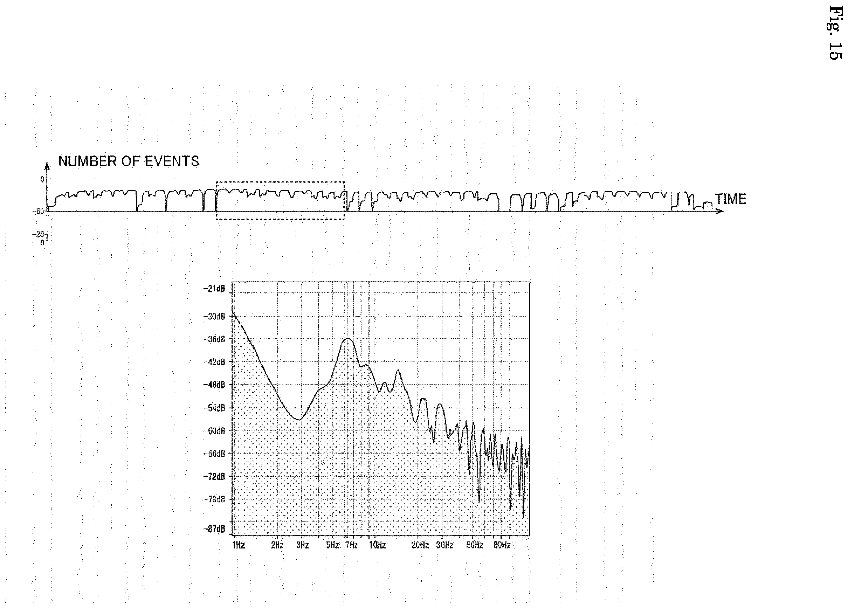

   Fig. 15 is an explanatory drawing showing a change of the number of events of type A and frequency components.
[Fig. 16]

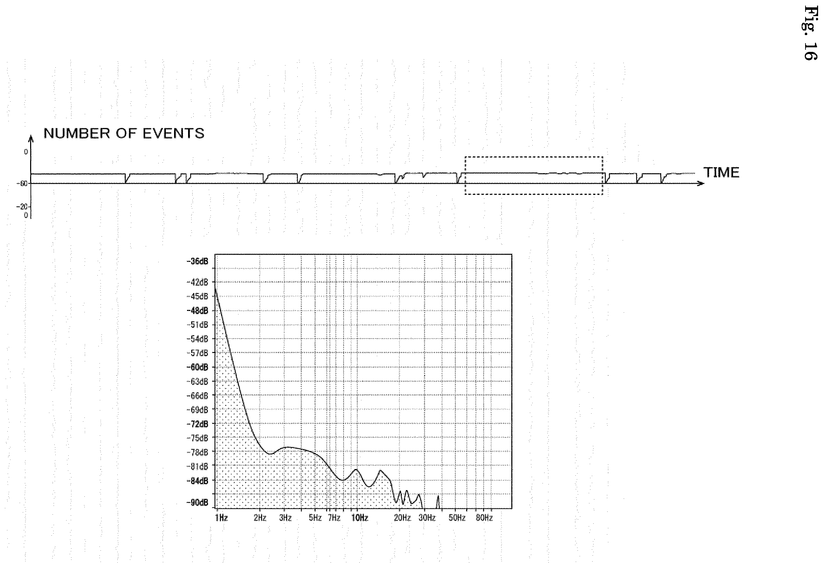

   Fig. 16 is an explanatory drawing showing a change of the number of events of type C and frequency components.
[Fig. 17]

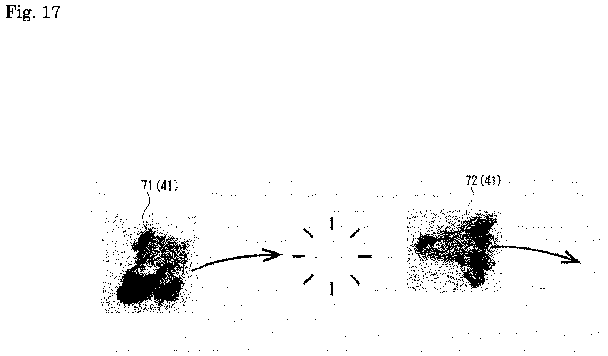

   Fig. 17 is an explanatory drawing of cluster aggregation.
[Fig. 18]

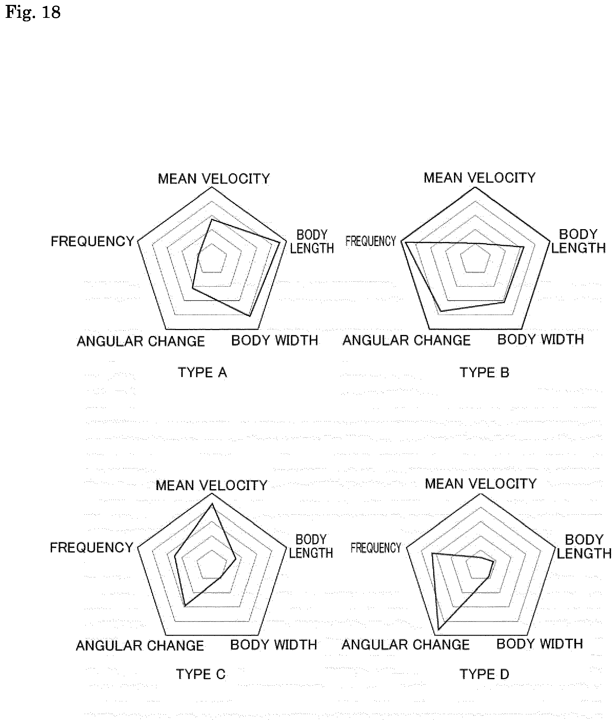

   Fig. 18 is an explanatory drawing of feature amounts of each type.
[Fig. 19]

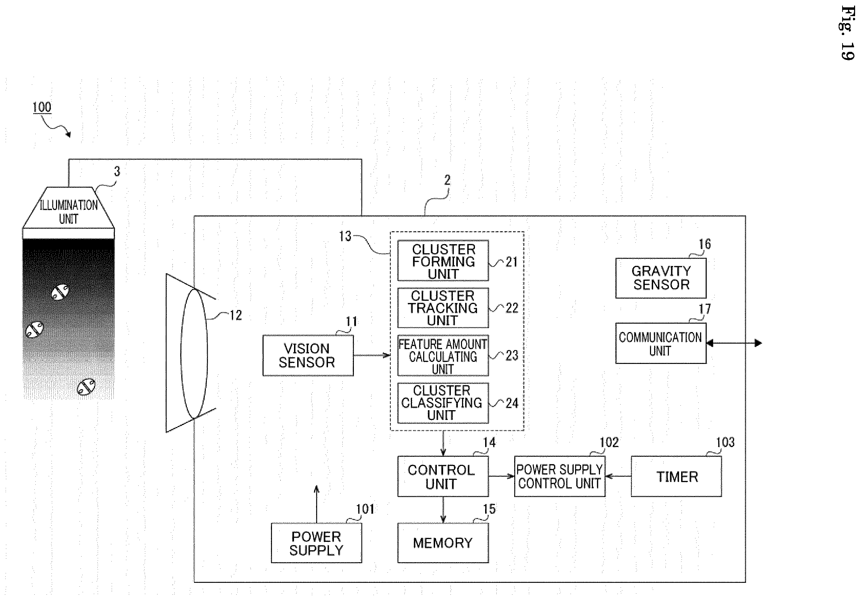

   Fig. 19 is an explanatory drawing illustrating the configuration of a measurement device according to usage example 1.
[Fig. 20]

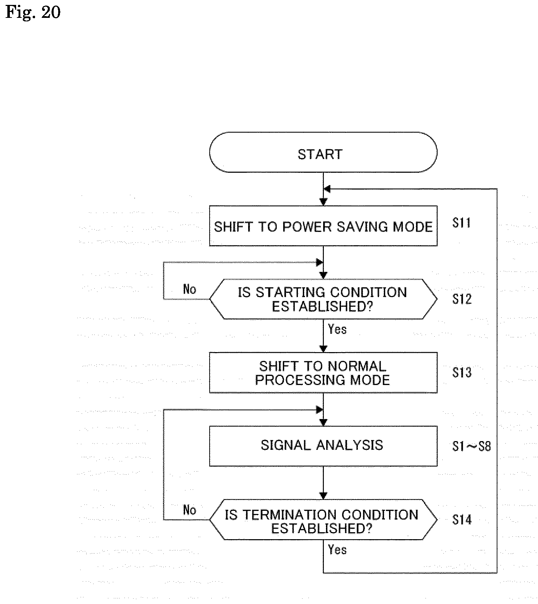

   Fig. 20 is a flowchart showing the processing flow of usage example 1.
[Fig. 21]

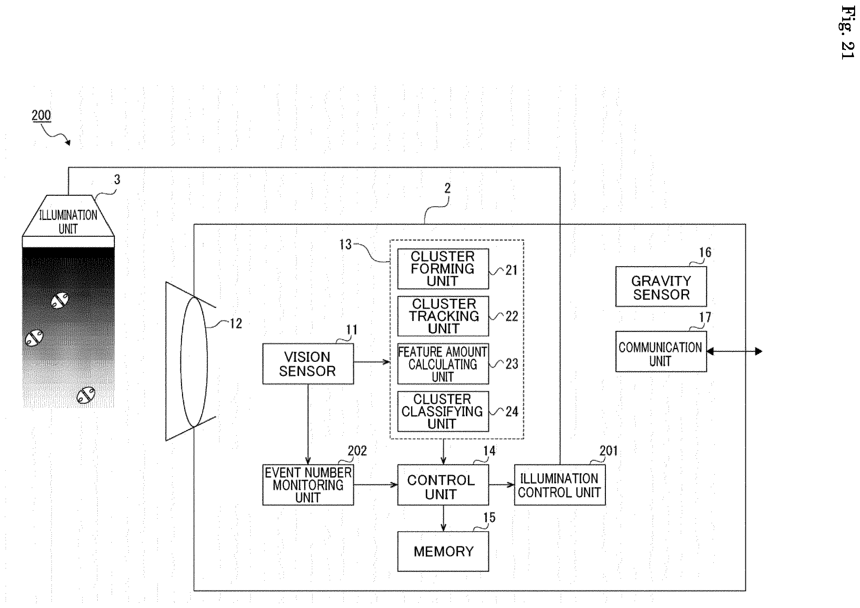

   Fig. 21 is an explanatory drawing illustrating the configuration of a measurement device according to usage example 2.
[Fig. 22]

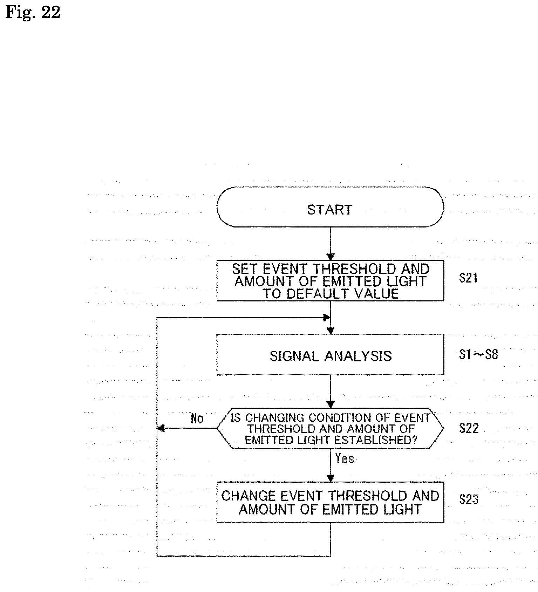

   Fig. 22 is a flowchart showing the processing flow of usage example 2.
[Fig. 23]

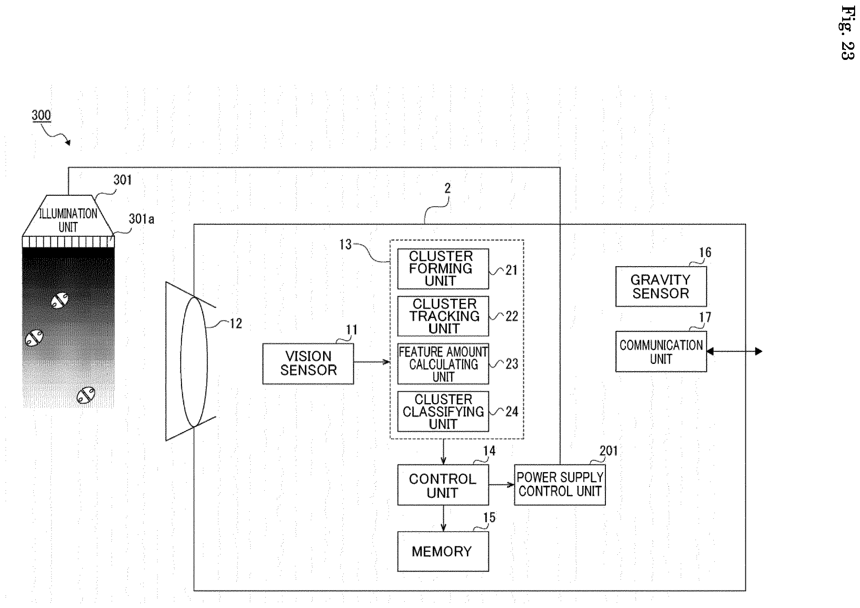

   Fig. 23 is an explanatory drawing illustrating the configuration of a measurement device according to usage example 3.
[Fig. 24]

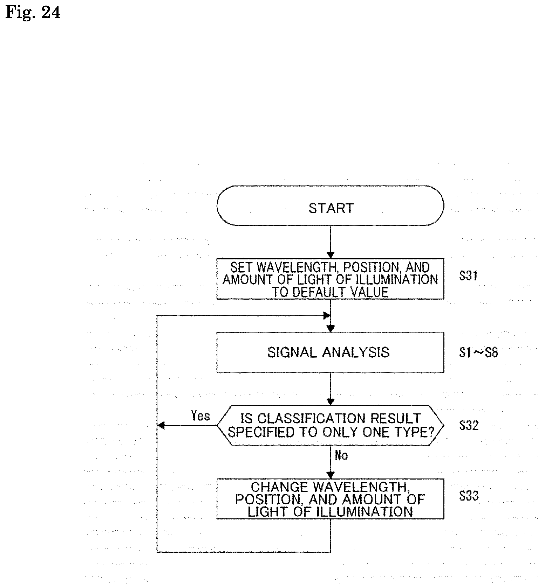

   Fig. 24 is a flowchart showing the processing flow of usage example 3.
[Fig. 25]

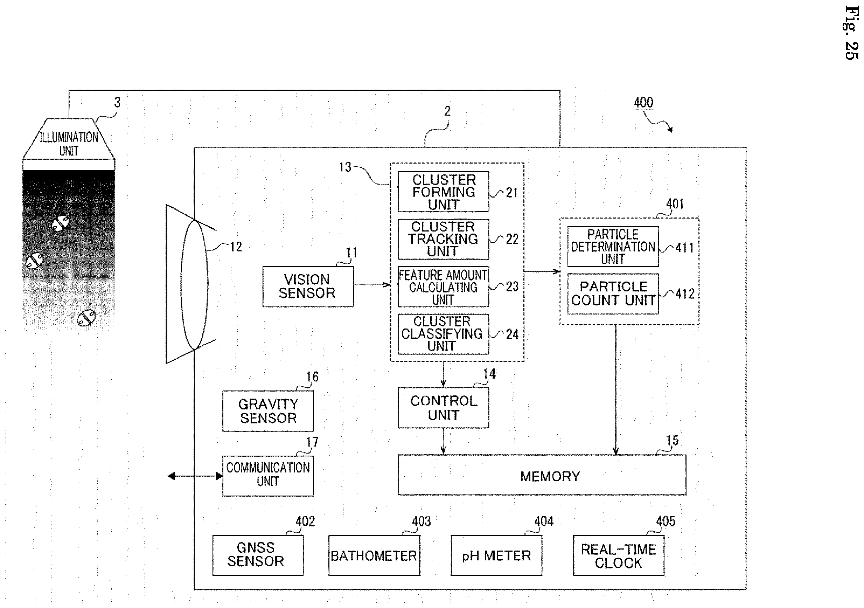

   Fig. 25 is an explanatory drawing illustrating the configuration of a measurement device according to usage example 4.
[Fig. 26]

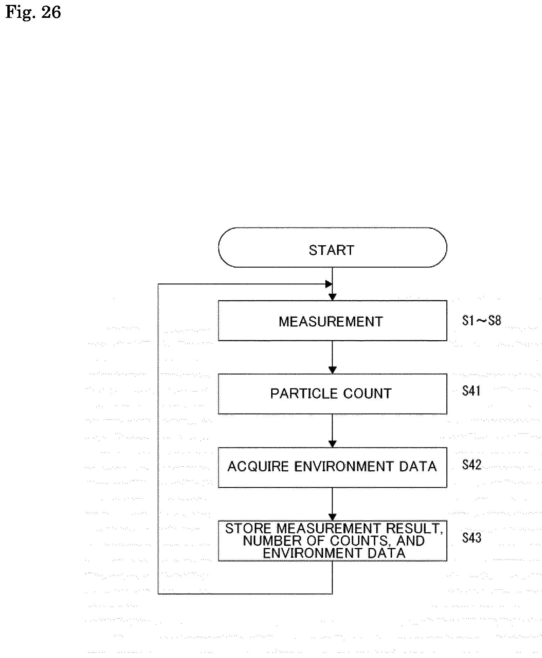

   Fig. 26 is a flowchart showing the processing flow of usage example 4.
[Fig. 27]

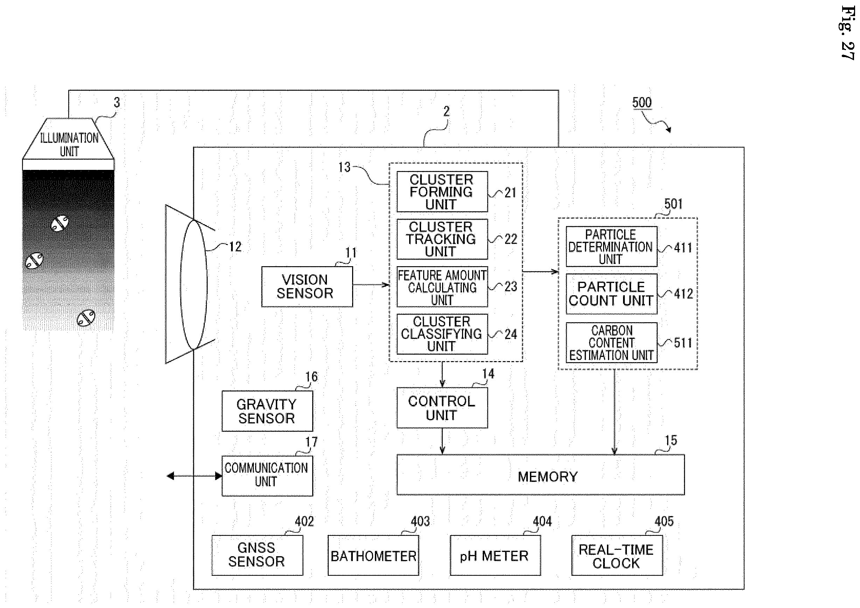

   Fig. 27 is an explanatory drawing illustrating the configuration of a measurement device according to usage example 5.

### [Description of Embodiments]

Hereinafter, an embodiment will be described in the following order.
<1. Configuration of Measurement Device>
<2. Signal Analysis>
<3. Cluster Formation>
<4. Cluster Tracking>
<5. Feature Amount Calculation>
<6. Cluster Aggregation>
<7. Cluster Classification>
<8. Usage Example>
<9. Another Configuration Example of Measurement Device>
<10. Summary of Embodiment>
< 11. Present Technique>

### <1. Configuration of Measurement Device>

The configuration of a measurement device 1 as an embodiment of the present technique will be described first.

The measurement device 1 is a device that measures organisms and non-living objects under water, for example, under the sea and measures target particles by calculating the feature amount of the target particles. The measurement is a concept including any one of the classification of types of the target particles, the calculation of the number and feature amount of target particles, and the recording or storage of a captured image of the target particles.

In this case, organisms serving as target particles are aquatic microorganisms such as phytoplankton, zooplankton, and juvenile aquatic organisms under water. Moreover, non-living objects serving as target particles are microplastics, dust, sand, marine snow, and air bubbles or the like. The target particles are merely exemplary and may be other objects.

Alternatively, the target particles may be particles moving spontaneously (hereinafter referred to as active particles) like zooplankton and juvenile aquatic organisms. Similarly, the target particles may be particles not moving spontaneously (hereinafter referred to as passive particles) like phytoplankton, microplastics, dust, sand, marine snow, and air bubbles or the like.

Fig. 1 is an explanatory drawing of the configuration of the measurement device 1. As illustrated in Fig. 1, the measurement device 1 includes a body unit 2 and an illumination unit 3. The illumination unit 3 may be provided in the body unit 2.

The body unit 2 includes a vision sensor 11, a lens 12, a signal analysis unit 13, a control unit 14, a memory 15, a gravity sensor 16, and a communication unit 17.

Fig. 2 is an explanatory drawing of the configuration of the vision sensor 11. Fig. 3 is an explanatory drawing of the outline of an event signal output from the vision sensor 11.

The vision sensor 11 is a sensor called a DVS (Dynamic Vision Sensor) or an EVS (Event-Based Vision Sensor). The vision sensor 11 detects an event occurring in a predetermined range under water through the lens 12. The event implies that a luminance change of incident light exceeds a certain ratio.

As illustrated in Fig. 2, the vision sensor 11 includes a plurality of pixels arranged in a two-dimensional array, the pixel including a photodiode 31, a voltage converter circuit 32, an asynchronous difference detection circuit 33, and a comparator 34. When being irradiated with incident light, the photodiode 31 generates a current proportionate to the luminance of light by a photoelectric effect. The voltage converter circuit 32 converts the current generated by the photodiode 31 into a voltage. The asynchronous difference detection circuit 33 detects a difference between the voltage converted by the voltage converter circuit 32 and a reference voltage.

The comparator 34 outputs an event signal (represented as a black arrow in Fig. 2), which indicates the occurrence of a plus event, when a voltage difference exceeds a plus threshold. Moreover, the comparator 34 outputs an event signal (represented as a white arrow in Fig. 2), which indicates the occurrence of a minus event, when a voltage difference exceeds (falls below) a minus threshold.

The event signal includes information about the coordinates (x, y) of a pixel where an event has occurred, a time, and whether the event is positive or negative (plus or minus). Moreover, the plus threshold and the minus threshold are ordinarily set at a predetermined default value. In some cases, the value may be changed.

For example, as shown in Fig. 3, it is assumed that a minus event signal is output at time T1. At this point, a voltage corresponding to a current luminance is set as a reference voltage. This sets a line (indicated by a dot-and-dash line in Fig. 3) corresponding to a plus threshold on the plus side with respect to the reference voltage and a line (indicated by a broken line in Fig. 3) corresponding to a minus threshold on the minus side with respect to the reference voltage.

Thereafter, when the luminance of incident light falls below the line corresponding to the minus threshold at falling time T2, the vision sensor 11 outputs a minus event signal. At this point, a voltage corresponding to a current luminance is set as a reference voltage as at time T1, and lines corresponding to a plus threshold and a minus threshold are set.

Moreover, when the luminance of incident light falls below the line corresponding to the minus threshold at falling time T3, the vision sensor 11 outputs a minus event signal. At this point, a voltage corresponding to a current luminance is set as a reference voltage as at time T1, and lines corresponding to a plus threshold and a minus threshold are set.

Thereafter, when the luminance of incident light exceeds the line corresponding to the plus threshold at rising time T4, the vision sensor 11 outputs a plus event signal.

As described above, the vision sensor 11 is an asynchronous image sensor that detects an event for each pixel in real time.

In the vision sensor 11, an event signal is read out for a pixel where the occurrence of an event has been detected. This enables readout at much higher speeds than in a synchronous image sensor in which readout is performed on all pixels at a predetermined frame rate, thereby reducing the amount of data to be read as one frame.

Thus, in the measurement device 1, the movements of target particles can be more quickly detected by using the vision sensor 11. Furthermore, the vision sensor 11 can reduce power consumption as well as the amount of data.

The signal analysis unit 13 is configured with a microcomputer including, for example, a CPU (Central Processing Unit), a ROM (Read Only Memory), and a RAM (Random Access Memory).

The signal analysis unit 13 acts as a cluster forming unit 21, a cluster tracking unit 22, a feature amount calculating unit 23, and a cluster classifying unit 24 in the present embodiment.

The cluster forming unit 21 forms a point group of events based on identical target particles, as a cluster on the basis of an event signal input from the vision sensor 11 in a predetermined period.

The cluster tracking unit 22 tracks the cluster formed by the cluster forming unit 21.

The feature amount calculating unit 23 calculates the feature amount of the cluster that is formed by the cluster forming unit 21 and is tracked by the cluster tracking unit 22.

The cluster classifying unit 24 classifies the cluster that is formed by the cluster forming unit 21 and is tracked by the cluster tracking unit 22, on the basis of the feature amount calculated by the feature amount calculating unit 23. In other words, the cluster classifying unit 24 classifies the type of target particles formed as the cluster.

The cluster forming unit 21, the cluster tracking unit 22, the feature amount calculating unit 23, and the cluster classifying unit 24 will be specifically described later.

The control unit 14 is configured with a microcomputer including, for example, a CPU, a ROM, and a RAM and controls the overall measurement device 1. The control unit 14 also performs processing for reading out data stored in the memory 15, processing for storing data in the memory 15, and transmission and reception of various types of data to and from external devices through the communication unit 17. The signal analysis unit 13 and the control unit 14 may be configured by identical hardware.

The memory 15 is configured with nonvolatile memory. The gravity sensor 16 detects a gravitational acceleration (a gravity direction) and outputs the result of detection to the control unit 14. The communication unit 17 conducts wire or radio data communications with external devices. The measurement device 1 does not necessarily include the gravity sensor 16.

The illumination unit 3 irradiates the imaging range of the vision sensor 11 with light. The illumination unit 3 allows illumination while switching light at different wavelengths, for example, emitting light at wavelengths of 10-nm intervals.

### <2. Signal Analysis>

Signal analysis (measurement method) for classifying the types of target particles on the basis of the event signal will be described below. Fig. 4 is a flowchart for describing a flow of signal analysis.

As shown in Fig. 4, at the start of signal analysis, the cluster forming unit 21 in step S1 acquires an event signal input from the vision sensor 11. As described above, the vision sensor 11 is configured to output an event signal when an event is detected for each pixel, so that the cluster forming unit 21 acquires an event signal anytime for each pixel where an event has occurred.

In step S2, the cluster forming unit 21 performs cluster formation such that a point group of events based on identical target particles is formed as a cluster at predetermined intervals (e.g., at 1-ms intervals) on the basis of the event signal acquired in step S1. The cluster formation will be specifically described later.

In step S3, the cluster tracking unit 22 performs cluster tracking such that the cluster formed by the cluster forming unit 21 is tracked at predetermined intervals (e.g., at 1-ms intervals). The cluster tracking will be specifically described later.

In step S4, the feature amount calculating unit 23 performs feature amount calculation such that a feature amount is calculated for the cluster that is formed by the cluster forming unit 21 and is tracked by the cluster tracking unit 22. The feature amount calculation will be specifically described later.

In step S5, the cluster tracking unit 22 performs cluster aggregation such that a plurality of different clusters of identical target particles are aggregated into a single cluster when the plurality of clusters are detected. The cluster aggregation will be specifically described later.

In step S6, the feature amount calculating unit 23 determines whether the clusters have been aggregated in step S5. If the clusters are aggregated (Yes at step S6), the feature amount calculating unit 23 calculates a feature amount for the aggregated clusters in step S7. The feature amount calculation of step S7 is the same processing as the feature amount calculation of step S4.

If the feature amount calculation is performed in step S7 or if it is determined that the clusters have not been aggregated in step S6 (No at step S6), the cluster classifying unit 24 in step S8 classifies the types of target particles for the clusters on the basis of the feature amount calculated in step S4 or step S7. The cluster classification will be specifically described later.

### <3. Cluster Formation>

Cluster formation will be described below. Fig. 5 is an explanatory drawing of a captured image based on the event signal. Fig. 6 is an explanatory drawing of an event in a captured image. In Fig. 6, multiple grids represent the pixels of a captured image.

In the vision sensor 11, an event signal is read out for a pixel where the occurrence of an event has been detected. Thus, as illustrated in Fig. 5, if a captured image of one frame is generated on the basis of an event signal acquired in a predetermined time, point groups of events based on identical particles are imaged as clusters 41. In addition to the clusters 41, multiple events are imaged as noise.

Thus, the cluster forming unit 21 first removes events other than events (effective events) based on target particles, that is, events as noise.

Specifically, as shown in Fig. 6, the cluster forming unit 21 determines whether an event 51 (painted black in Fig. 6) based on, for example, the latest event signal acquired in 1 ms is surrounded by a predetermined number of events 52 (hatched in Fig. 6) based on, for example, a preceding event signal acquired in 3 ms. In this case, the predetermined number is set at a value that allows a determination on whether the event 51 is noise or a part of the cluster 41.

If the number of events 52 is equal to or larger than the predetermined number around the event 51, the cluster forming unit 21 determines that the event 51 is an effective event. If the number of events 52 is smaller than the predetermined number around the event 51, the cluster forming unit 21 determines that the event 51 is an ineffective event, that is, noise.

As described above, the cluster forming unit 21 removes noise at predetermined intervals (e.g., at 1-ms intervals) from the event 51 based on the event signal acquired from the vision sensor 11, and sets only an effective event as an event to be processed downstream.

Moreover, the cluster forming unit 21 stores, in a predetermined buffer of RAM, an event signal determined as an effective event with a predetermine period (e.g., 100 ms). Therefore, the cluster forming unit 21 deletes the oldest 1-ms event signal from the predetermined buffer when storing the latest 1-ms event signal in the predetermined buffer.

For an event based on the event signal stored in the predetermined buffer, the cluster forming unit 21 then forms a group of events in a predetermined range (e.g., in two surrounding pixels) as events based on identical target particles, so that the single cluster 41 is formed.

### <4. Cluster Tracking>

When the event signal stored in the predetermined buffer is updated at predetermined intervals (e.g., at 1-ms intervals), the cluster tracking unit 22 performs cluster tracking by updating the cluster 41 on the basis of the updated event signal.

Specifically, if an event included in the cluster 41 is based on the oldest event signal to be deleted from the predetermined buffer, the cluster tracking unit 22 removes the event from the cluster 41.

Moreover, if an event based on the latest event signal is placed in a predetermined range of any one of events constituting the cluster 41, the cluster tracking unit 22 adds the event to the cluster 41.

Thus, the cluster tracking unit 22 can always form the cluster 41 including only the latest event. The cluster tracking unit 22 can also track the cluster 41 moving according to a movement of target particles.

### <5. Feature Amount Calculation>

The feature amount calculating unit 23 calculates the feature amount of the cluster 41 tracked by the cluster tracking unit 22. The feature amount may be related to a movement, a frequency, an event, or a shape.

A movement-related feature amount is, for example, a mean velocity, a body length, a body width, a frequency of an angular change, a body length ratio to a mean velocity, a body length ratio to a maximum instantaneous velocity, directional change sustainability, the presence or absence of taxis, positive and negative taxis, a frequency of stop, longitudinal mobility, lateral mobility, the number of times or frequency of a rapid change of velocity, uniform velocity sustainability, temporal transitional dispersion of a velocity absolute value, values of temporal transitional dispersion of absolute values of velocity components in horizontal and vertical directions or the sum of the values, a rotation arc width, a twisting motion period, velocity dispersion, or a maximum instantaneous acceleration of the cluster 41. The taxis includes, for example, phototaxis, thermotaxis, chemotactic, haptotaxis, barotaxis, and electrotaxis. A frequency-related feature amount is, for example, a frequency of fluctuations (change) in the number of events, a frequency of fluctuations in size change, the number of frequency peaks or a peak value as a result of frequency analysis of a temporal change of an increase in the number of events regarding plus events and minus events or both of the plus and minus events, or the number of frequency peaks or a peak value as a result of frequency analysis of a temporal change or a locus of the gravity (coordinate mean value) of events regarding plus events and minus event or both of the plus and minus events.

An event-related feature amount is, for example, a ratio of plus events and minus events or an increase rate of events.

A shape-related feature amount is, for example, a scale ratio in the vertical and horizontal directions, a variation range of a scale ratio in the vertical and horizontal directions, an event ratio near a head and a tail in the presence or absence of antennae or the like projecting from the head, dispersion of coordinate values of plus events and minus events, or dispersion of distances between centroids (coordinate mean value) of plus events and minus events.

As described above, the cluster 41 may have various feature amounts. The feature amount calculating unit 23 calculates at least one of feature amounts classified as a movement-related feature amount, a frequency-related feature amount, an event-related feature amount, and a shape-related feature amount of the cluster 41. In this case, five feature amounts calculated as an example are a mean velocity, a body length, a body width, a frequency of an angular change of the cluster 41, and a frequency of fluctuations in the number of events (hereinafter simply referred as a frequency).

The feature amount calculating unit 23 calculates, at predetermined intervals (e.g., at 4-ms intervals), a mean velocity, a body length, a body width, a frequency of an angular change of the cluster 41, and a frequency as feature amounts of the cluster 41 tracked in cluster tracking.

### [5-1. Mean Velocity]

Fig. 7 is an explanatory drawing of calculation of a mean velocity of the cluster. As shown in Fig. 7, the feature amount calculating unit 23 calculates a distance of travel (the number of pixels) from a starting point 61 to an end point 62 of the cluster 41 tracked by cluster tracking. The starting point 61 is calculated as the center position (the center in the vertical and horizontal directions) of the initially formed cluster 41. The end point 62 is calculated as the center position of the current cluster 41. Moreover, the total distance of a travel route 63 from the starting point 61 to the end point 62 is calculated as a travel distance.

The feature amount calculating unit 23 then calculates a rate of travel by dividing the calculated travel distance by a time of travel. The feature amount calculating unit 23 also calculates a mean velocity by calculating the mean value of a rate of travel of the same cluster 41. The rate of travel is calculated at predetermined intervals.

Fig. 8 shows a mean velocity for each type. Fig. 8 shows the measurement results of mean velocities for four different types of organisms (types A to D). In Fig. 8, the vertical axes and horizontal axes of four graphs are shown with the same scale.

As shown in Fig. 8, type A indicates a distribution with relatively low mean velocities. Type B shows mean values uniformly distributed from a low velocity to a high velocity. Type C shows mean velocities relatively lower than those of type A. Type D shows mean velocities relatively lower than those of types A, B, and C and substantially no variations in mean value among individuals.

As described above, the distributions of mean velocities vary among types. The feature amount calculating unit 23 calculates a mean velocity as a feature amount, so that the accuracy of classification can be improved by using a mean velocity in cluster classification by the cluster classifying unit 24, which will be described later.

### [5-2. Body Length]

Fig. 9 is an explanatory drawing showing calculation of a body length of the cluster. As shown in Fig. 9, the feature amount calculating unit 23 sets a rectangular bounding box 64 around the cluster 41 tracked by the cluster tracking. The feature amount calculating unit 23 then calculates a length (X) in the horizontal direction (lateral direction) and a length (Y) in the vertical direction (longitudinal direction) of the bounding box 64.

Thereafter, the feature amount calculating unit 23 sets longer one of the calculated length (X) in the horizontal direction and the calculated length (Y) in the vertical direction as a temporary body length. The feature amount calculating unit 23 also calculates the mean value of a temporary body length of the same cluster 41, the temporary body length being calculated at predetermined intervals, so that the mean value is obtained as the body length of the cluster 41.

Fig. 10 is an explanatory drawing of body lengths for each type. Fig. 10 shows the measurement results of body lengths for four different types of organisms (types A to D) as in Fig. 8. In Fig. 10, the vertical axes and horizontal axes of four graphs are shown with the same scale.

As shown in Fig. 10, type A indicates a wide distribution of body lengths ranging from a relatively short body length to a long body length. Type B indicates a relatively wide distribution of body lengths, many of which are longer than those of type A. Type C indicates a narrow distribution of body lengths that are shorter than those of types A and B. Type D indicates body lengths shorter than those of types A, B, and C and substantially no variations in body length among individuals.

As described above, the distributions of body lengths vary among types. The feature amount calculating unit 23 calculates a body length as a feature amount, so that the accuracy of classification can be improved by using a body length in cluster classification by the cluster classifying unit 24, which will be described later.

### [5-3. Body Width]

Fig. 11 is an explanatory drawing showing calculation of a body width of the cluster. As shown in Fig. 11, the feature amount calculating unit 23 calculates a traveling-direction vector 65 of the tracked cluster 41 tracked by the cluster tracking. Specifically, the feature amount calculating unit 23 calculates, as the traveling-direction vector 65, a vector connected from the center position of the cluster 41 preceding at, e.g., 100 ms to the center position of the current (latest) cluster 41.

The feature amount calculating unit 23 also calculates a unit vector orthogonal to the traveling-direction vector 65, as an orthogonal unit vector 66.

The feature amount calculating unit 23 extracts, as target events 67, any multiple (e.g., 16) events from latest (most recently at 1 ms) events among events constituting the cluster 41. The feature amount calculating unit 23 then calculates, as a body-width vector 68, a vector connected from the center position of the current (latest) cluster 41 to the target event 67.

Thereafter, the feature amount calculating unit 23 calculates, for each of the target events 67, a length orthogonal to the traveling direction of the cluster 41 by obtaining the inner product of the orthogonal unit vector 66 and the body-width vector 68.

The feature amount calculating unit 23 sets, as a temporary body width, longest one of lengths orthogonal to the traveling direction, the lengths being calculated for the target events 67. The feature amount calculating unit 23 also calculates the mean value of a temporary body width of the same cluster 41, the temporary body width being calculated at predetermined intervals, so that the mean value is obtained as the body width of the cluster 41.

Fig. 12 is an explanatory drawing of body widths for each type. Fig. 11 shows the measurement results of body widths for four different types of organisms (types A to D) as in Figs. 8 and 10. In Fig. 12, the vertical axes and horizontal axes of four graphs are shown with the same scale.

As shown in Fig. 12, type A indicates a wide distribution of body widths ranging from a relatively small body width to a large body width. Type B indicates a relatively wide distribution of body lengths, many of which are longer than those of type A. Type C indicates a narrow distribution of body lengths that are shorter than those of types A and B. Type D indicates body lengths shorter than those of types A, B, and C and substantially no variations in body length among individuals.

As described above, the distributions of body widths vary among types. The feature amount calculating unit 23 calculates a body width as a feature amount, so that the accuracy of classification can be improved by using a body width in cluster classification by the cluster classifying unit 24, which will be described later.

### [5-4. Frequency of Angular Change]

Fig. 13 is an explanatory drawing showing calculation of a frequency of an angular change. As shown in Fig. 13, as in the calculation of the body width, the feature amount calculating unit 23 calculates the traveling-direction vector 65 of the cluster 41 tracked by the cluster tracking and stores the traveling-direction vector 65 in the RAM. Moreover, the feature amount calculating unit 23 reads the traveling-direction vector 65 preceding at, for example, 100 ms from the RAM and sets the vector as a forward traveling-direction vector 69.

The feature amount calculating unit 23 also calculates the inner product of the unit lengths of the traveling-direction vector 65 and the forward traveling-direction vector 69, that is, an angle 70 formed by the traveling-direction vector 65 and the forward traveling-direction vector 69.

The feature amount calculating unit 23 also counts the number of times when the angle 70 formed by the traveling-direction vector 65 and the forward traveling-direction vector 69 falls within the range of a predetermined angle, and sets the count value as the frequency of an angular change. The predetermined angle is set at an angle where target particles have changed the directions.

Fig. 14 is an explanatory drawing of a frequency of an angular change for each type. Fig. 14 shows the measurement results of a frequency of an angular change for four different types of organisms (types A to D) as in Figs. 8, 10, and 12. In Fig. 14, the vertical axes and horizontal axes of four graphs are shown with the same scale.

As shown in Fig. 14, type A indicates a distribution of relatively low frequencies of an angular change. Type B indicates a distribution of higher frequencies of an angular change than those of type A. Type C indicates a wide distribution of lower frequencies to higher frequencies of an angular change than those of type A. Type D indicates a narrow distribution of a higher frequency of an angular change than those of types A, B, and C.

As described above, the distributions of frequencies of an angular change vary among types. The feature amount calculating unit 23 calculates a frequency of an angular change as a feature amount, so that the accuracy of classification can be improved by using a frequency of an angular change in cluster classification by the cluster classifying unit 24, which will be described later.

### [5-5. Frequency]

Fig. 15 is an explanatory drawing showing a change of the number of events of type A and frequency components. Fig. 16 is an explanatory drawing showing a change of the number of events of type C and frequency components.

If target particles are organisms, the number of events detected by the vision sensor 11 changes according to the way target particles move (swim). This is because the natatorial organs of target objects move at a predetermined frequency. Hence, the number of events changes at a predetermined frequency depending upon the type of target particles.

As shown in the upper part of Fig. 15, when the number of events is measured for type A, the number of events changes in a specific pattern. Thus, in frequency analysis on a part surrounded by a broken line in the upper part of Fig. 15, frequency components at about 7 Hz are detected as a peak as shown in the lower part of Fig. 15. In other words, it is understood that type A causes natatorial organs to move at about 7 Hz.

As shown in the upper part of Fig. 16, when the number of events is measured for type C, the number of events has a substantially constant value while the organisms of type C move. In frequency analysis on a part surrounded by a broken line in the upper part of Fig. 16, a frequency having a specific peak is not detected as shown in the lower part of Fig. 16.

Thus, the feature amount calculating unit 23 performs frequency analysis for the number of events of the cluster 41 and calculates a peak frequency. As described above, a peak frequency may be uncalculated.

Thus, values indicated at frequencies vary among types. The feature amount calculating unit 23 calculates a frequency distribution as a feature amount, so that the accuracy of classification can be improved by using a frequency in cluster classification by the cluster classifying unit 24, which will be described later.

### <6. Cluster Aggregation>

As described above, the cluster 41 formed by the cluster forming unit 21 is tracked by the cluster tracking unit 22. However, the vision sensor 11 outputs an event signal if the luminance of incident light changes. Thus, the event signal is not output when, for example, organisms serving as target particles stop. Hence, if organisms serving as target particles stop, the cluster 41 cannot be tracked by the cluster tracking unit 22.

When the stopped organisms start moving again, another cluster 41 for the organisms is formed by the cluster forming unit 21. In other words, even if the cluster 41 before the stop and the cluster 41 after the movement are formed and tracked on the basis of the same organisms, the clusters 41 are detected as different clusters.

Moreover, if multiple organisms overlap each other in the imaging range of the vision sensor 11, the clusters 41 based on the organisms may be combined or separated, leading to difficulty in tracking the clusters 41 as the same organisms.

The cluster tracking unit 22 performs cluster aggregation, so that a plurality of different clusters 41 of identical target particles are aggregated into the single cluster 41.

Fig. 17 is an explanatory drawing of cluster aggregation. Specifically, as shown in Fig. 17, the cluster tracking unit 22 compares a feature amount of the cluster 41 lost in sight (hereinafter referred to as a lost cluster 71) with a feature amount of the cluster 41 additionally generated (hereinafter referred to as a new cluster 72) in a predetermined time in a predetermined range from a position where the lost cluster 71 is missed (indicated by concentrated lines).

For example, the cluster tracking unit 22 obtains, as the new cluster 72, the cluster 41 generated in a predetermined time in a predetermined range from a position where the lost cluster 71 is missed. The cluster tracking unit 22 then compares the feature amounts of the lost cluster 71 and the new cluster 72. If the feature amounts fall within a range of identical target particles, the lost cluster 71 and the new cluster 72 are aggregated into the single cluster 41.

When the clusters 41 are aggregated, the feature amount calculating unit 23 recalculates the feature amount of the aggregated clusters 41. In this case, for example, a mean velocity, a body length, a body width, a frequency of an angular change, and a frequency of the cluster 41 are recalculated.

As described above, if identical target particles are detected as different clusters 41, the aggregation of the clusters 41 allows a feature amount to be calculated by using information with an extended time, thereby improving the accuracy of calculation of a feature amount.

Moreover, identical target particles are detected as the single cluster 41, thereby improving calculation accuracy in the calculation of the number of target particles.

### <7. Cluster Classification>

When a feature amount is calculated for the cluster 41, the cluster classifying unit 24 classifies the cluster 41, that is, specifies the type of target particles on the basis of the calculated feature amount. In this case, the type of target particles may be specified in a rule-based manner or the type of target particles may be specified by machine learning.

Fig. 18 is an explanatory drawing of feature amounts of each type. For example, if the type of target particles is specified in a rule-based manner, as shown in the example of Fig. 18, the range of feature amounts is set for each type to be specified. Information about the type is stored in the ROM or the memory 15. If the feature amounts of the cluster 41 are included in the range of feature amounts of a specific type, the cluster classifying unit 24 specifies the cluster 41 as the specific type.

If the type of target particles is specified by machine learning, a learning model is generated, which has learned the relationship between known feature amounts for each type and the types as teacher data, and the learning model is stored in the ROM or the memory 15. The cluster classifying unit 24 then specifies the type of the cluster 41 by introducing the feature amounts of the cluster 41 into the learning model.

Moreover, calculated feature amounts (e.g., a body length and a body width) may have different values for the same type because of the metamorphosis period or the growth process. Hence, the cluster classifying unit 24 comprehensively specifies the type according to various feature amounts.

As described above, the signal analysis unit 13 forms and tracks the cluster 41 on the basis of the event signal input from the vision sensor 11 and calculates the feature amount of the cluster 41. The signal analysis unit 13 then specifies the type of target particles on the basis of the feature amount of the cluster 41. Moreover, the control unit 14 stores specified information in the memory 15. At this point, the control unit 14 may store the specified information in relation to external environment information. The external environment information may be, for example, depth, position coordinates (latitude and longitude of a measurement point), electrical conductivity, a temperature, ph, the concentration of gas (e.g., methane, hydrogen, or helium), the concentration of metal (e.g., manganese or iron), and the flow velocity of peripheral liquid.

### <8. Usage Example>

Hereinafter, usage examples 1 to 5 will be described as usage examples of the measurement device 1. In usage examples 1 to 5, the same configurations as the measurement device 1 of the embodiment are denoted by the same reference numerals, and descriptions thereof are omitted. The usage examples 1 to 5 may be independently used or two or more of the usage examples may be used in combination.

### [8-1. Usage Example 1]

Fig. 19 is an explanatory drawing of the configuration of a measurement device 100 according to usage example 1. In usage example 1, signal analysis in the measurement device 100 is interrupted or reduced to achieve low power consumption. The measurement device 100 is kept under the sea for several months to several years and thus low power consumption can extend the measurement period.

As illustrated in Fig. 19, the measurement device 100 includes a power supply 101, a power supply control unit 102, and a timer 103 in addition to the configuration of the measurement device 1.

The power supply 101 is a battery in which power to be supplied to each unit is stored. The power supply control unit 102 is configured with a computer including a CPU, a RAM, and a ROM and controls power to be supplied from the power supply 101 to each unit. The power supply control unit 102 may be configured with the same hardware as the signal analysis unit 13 or the control unit 14. The timer 103 outputs a signal as a trigger to the power supply control unit 102 at regular intervals.

Fig. 20 is a flowchart showing the processing flow of usage example 1. As shown in Fig. 20, when the processing is started, the power supply control unit 102 shifts the measurement device 100 to a power saving mode in step S11. In the power saving mode, power supply from the power supply 101 is controlled so as to stop or reduce (intermittently supply) power to the units (e.g., the vision sensor 11, the signal analysis unit 13, the control unit 14, and the gravity sensor 16) of the measurement device 100. The measurement device 100 is placed into the power saving mode, so that signal analysis can be interrupted or reduced.

Thereafter, in step S12, the power supply control unit 102 determines whether the starting conditions have been established or not. In this case, the set starting conditions are the entry of a signal as a trigger from the timer 103, the entry of event signals from the vision sensor 11 operating in the power saving mode such that the number of event signals is equal to or larger than a predetermined value, and the entry of a command for a starting instruction from the outside through the communication unit 17.

Step S12 is repeated until the starting conditions are established. When the starting conditions are established (Yes at step S12), the power supply control unit 102 shifts the measurement device 100 to a normal processing mode in step S13. In the normal processing mode, power supply from the power supply 101 is controlled so as to always supply power to the units (e.g., the vision sensor 11, the signal analysis unit 13, the control unit 14, and the gravity sensor 16) of the measurement device 100.

Thereafter, the signal analysis of steps S1 to S8 is performed, and then the power supply control unit 102 determines whether the termination condition has been established or not in step S14. In this case, the termination condition is at least one of the entry of a signal as a trigger from the timer 103, the establishment of predetermined conditions based on the number or feature amounts of the clusters 41, the number or feature amounts being calculated in the signal analysis, and the entry of a command for a termination instruction from the outside through the communication unit 17.

Measurement (steps S1 to S8) is conducted until the termination condition is established. When the termination condition is established (Yes at step S14), the process returns to step S11 and the power supply control unit 102 shifts the measurement device 100 to the power saving mode.

As described above, the power supply control unit 102 switches the modes on the basis of, for example, the number or feature amounts of the clusters, achieving low power consumption and efficient measurement of target particles.

### [8-2. Usage Example 2]

Fig. 21 is an explanatory drawing of the configuration of a measurement device 200 according to usage example 2. For example, if target particles in the measurement range of the vision sensor 11 are sufficiently large in size and actively move, the power consumption of the vision sensor 11 and the signal analysis unit 13 may be increased when the number of event signals obtained from target particles is equal to or larger than the number of event signals required by the signal analysis unit 13. If target particles move slowly, the required number of events cannot be provided and thus correct measurement results cannot be obtained.

Thus, in usage example 2, if the number of events detected by the vision sensor 11 is extremely large or small, the event threshold (plus threshold, minus threshold) of the vision sensor 11 or the amount of light emitted from the illumination unit 3 is adjusted to optimize the number of events.

As illustrated in Fig. 21, the measurement device 200 includes an illumination control unit 201 and an event number monitoring unit 202 in addition to the configuration of the measurement device 1.

The illumination control unit 201 is configured with a computer including a CPU, a RAM, and a ROM and controls the amount of light emitted from the illumination unit 3. The event number monitoring unit 202 is configured with a computer including a CPU, a RAM, and a ROM and monitors the number of event signals output from the vision sensor 11, that is, the number of events obtained by the vision sensor 11. The illumination control unit 201 and the event number monitoring unit 202 may be configured with the same hardware as the signal analysis unit 13 or the control unit 14.

Fig. 22 is a flowchart showing the processing flow of usage example 2. As shown in Fig. 22, when the processing is started, the control unit 14 sets the event threshold (plus threshold, minus threshold) of the vision sensor 11 to a default value in step S21. Furthermore, the illumination control unit 201 sets, to a default value, the amount of light emitted from the illumination unit 3.

Thereafter, the signal analysis of steps S1 to S8 is performed. At this point, the event number monitoring unit 202 monitors the number of events. In step S22, the control unit 14 determines whether the changing condition of an event threshold and the amount of light has been established or not. In this case, the changing condition is at least one of the number of events equal to or larger than a first predetermined number or equal to or smaller than a second predetermined number, the ratio of failures in tracking of the cluster 41, the ratio being equal to or larger than a first predetermined value or equal to or smaller than a second predetermined value, and the cluster 41 having a predetermined size or larger with a sufficient momentum (a means velocity of predetermined value of larger) while an extremely large number of events may be provided. The first predetermined number is larger than the second predetermined number, and the first predetermined value is larger than the second predetermined value.

More specifically, the changing condition for a large number of events is at least one of the number of events equal to or larger than the first predetermined number, the ratio of failures in tracking of the cluster 41, the ratio being equal to or smaller than the second predetermined value, and the cluster 41 having a predetermined size or larger with a sufficient momentum while an extremely large number of events may be provided. Furthermore, the changing condition for a small number of events is at least one of the number of events equal to or smaller than the second predetermined number and the ratio of failures in tracking of the cluster 41, the ratio being equal to or larger than the first predetermined value.

If the changing condition is not established (No at step S22), the process returns to the signal analysis of steps S1 to S8. If the changing condition is established (Yes at step S22), the control unit 14 changes the event threshold (plus threshold, minus threshold) of the vision sensor 11 in step S23. Furthermore, the illumination control unit 201 changes the amount of light emitted from the illumination unit 3. In this case, if the changing condition for a large number of events is established, the event threshold is increased or the amount of light is reduced. If the changing condition for a small number of events is established, the event threshold is reduced or the amount of light is increased.

As described above, the measurement device 200 can reduce power consumption and accurately measure target particles by properly adjusting the number of events. When the changing conditions is established, the measurement device 200 may perform at least one of a change of the event threshold and a change of the amount of light emitted from the illumination unit 3 or both of the changes.

### [8-3. Usage Example 3]

Fig. 23 is an explanatory drawing of the configuration of a measurement device 300 according to usage example 3. In usage example 3, an illumination unit 301 includes a plurality of light sources 301a that can be separately turned on or off. The light sources 301a are turned on or off and wavelengths and light amounts are switched on the basis of the results of signal analysis.

As illustrated in Fig. 23, the measurement device 300 includes the illumination unit 301 instead of the illumination unit 3 of the measurement device 1 and includes the illumination control unit 201. The illumination unit 301 includes the plurality of light sources 301a that can be separately turned on or off. The illumination unit 301 can change a light irradiation position as a whole by turning on or off the plurality of light sources 301a.

Fig. 24 is a flowchart showing the processing flow of usage example 3. As shown in Fig. 24, when the processing is started, the illumination control unit 201 sets the wavelength, position, and amount of light emitted from the illumination unit 301, to default values in step S31.

Thereafter, the signal analysis of steps S1 to S8 is performed, and then in step S32, the control unit 14 determines whether the classification result of the specific cluster 41 has been specified to only one type on the basis of the result of signal analysis.

If the classification result has not been specified to only one type (No at step S32), in other words, classification candidates are specified for a plurality of types, the illumination control unit 201 in step S33 changes the wavelength, position, and amount of light emitted from the illumination unit 301 such that the classification of one type is specified. In this case, the light sources 301a are turned on or off and the wavelength, position, and amount of emitted light are changed according to, for example, the phototaxis of the type of the classification candidate. If the classification result is specified to only one type (Yes at step S32), the signal analysis (steps S1 to S8) is continued.

As described above, the measurement device 300 changes the wavelength, position, and amount of light emitted from the illumination unit 301, so that the classification accuracy of target particles can be improved by measurement using the phototaxis of target particles as another feature amount. Moreover, the targets of measurement can be narrowed down, for example, only target particles of one specific type can be selected to be irradiated with light and measured, achieving efficient measurement.

Also when predetermined conditions other than classification candidates specified for the plurality of types are established, the illumination control unit 201 may change the wavelength, position, and amount of light emitted from the illumination unit 301. Furthermore, the illumination control unit 201 may change only the position of light emitted from the illumination unit 301.

### [8-4. Usage Example 4]

Fig. 25 is an explanatory drawing of the configuration of a measurement device 400 according to usage example 4. In usage example 4, in addition to the result of signal analysis, external environment information measured by various sensors may be stored in relation to the result of signal analysis.

As illustrated in Fig. 25, the measurement device 400 includes a particle analysis unit 401, a GNSS sensor (Global Navigation Satellite System) 402, a bathometer 403, a pH meter 404, and a real-time clock 405 in addition to the configuration of the measurement device 100.

The particle analysis unit 401 is configured with a computer including a CPU, a RAM, and a ROM and includes a particle determination unit 411 and a particle count unit 412.

The particle determination unit 411 further classifies the target particles classified by the signal analysis, on the basis of preset conditions. For example, the particle determination unit 411 classifies, as active particles, target particles also moving upward and classifies, as passive particles, target particles only moving downward on the basis of the moving directions of target particles (cluster 41).

The particle count unit 412 counts the number of particles classified by the particle determination unit 411.

The particle analysis unit 401 may be configured with the same hardware as the signal analysis unit 13 or the control unit 14. The particle determination unit 411 and the particle count unit 412 may be provided in the cluster classifying unit 24.

The GNSS sensor 402 acquires position information on the measurement device 400. The bathometer 403 measures the depth of a position where the measurement device 400 is located. The pH meter 404 measures the pH value of a water area where the measurement device 400 is located. The real-time clock 405 is an integrated circuit having a clock function and outputs a current time.

Fig. 26 is a flowchart showing the processing flow of usage example 4. As shown in Fig. 26, when the processing is started, the signal analysis of steps S1 to S8 is performed, and then in step S41, the particle determination unit 411 further classifies target particles classified by the signal analysis, on the basis of the preset conditions. The particle count unit 412 counts the number of classified particles.

In step S42, the control unit 14 acquires measured or output values from the gravity sensor 16, the GNSS 402, the bathometer 403, the pH meter 404, and the real-time clock 405. In step S43, the control unit 14 stores, in the memory 15, the result of processing in step S41 in relation to the result of acquisition in step S42 as external environment information.

As described above, the measurement device 400 stores the number of active particles and passive particles in relation to various kinds of external environment information, allowing observation of, for example, the number and density of organisms and acquisition of the ratio of passive particles such as marine snow. Thus, an index for evaluating biodiversity and the degree of environmental pollution in the sea can be also found.

### [8-5. Usage Example 5]

Fig. 27 is an explanatory drawing of the configuration of a measurement device 500 according to usage example 5. In usage example 5, a carbon content on the sea bottom is estimated on the basis of the result of signal analysis.

As illustrated in Fig. 27, the measurement device 500 includes a particle analysis unit 501 instead of the particle analysis unit 401 in the measurement device 400 of usage example 4.

The particle analysis unit 501 is configured with a computer including a CPU, a RAM, and a ROM and includes the particle determination unit 411, the particle count unit 412, and a carbon content estimation unit 511.

The carbon content estimation unit 511 determines, as particles build up on the sea bottom, target particles classified as passive particles by the particle determination unit 411 and estimates a carbon content on the sea bottom on the basis of the type, feature amount (a feature amount indicating a size, e.g., a body length or a body width), and number of passive particles.

For example, a carbon content per unit size for each type classified as passive particles is stored in the memory 15. The carbon content estimation unit 511 estimates a carbon content according to the carbon content per unit size and the relationship between the size and the number of passive particles.

As described above, the measurement device 500 estimates a carbon content on the sea bottom, thereby acquiring an index for reducing greenhouse gas.

### <9. Another Configuration Example of Measurement Device>

The embodiment is not limited to the specific examples described above, and configurations as various modifications can be adopted.

The measurement device may include an imaging sensor. The imaging sensor is, for example, a CCD (Charge Coupled Device) or a CMOS (Complementary Metal-Oxide-Semiconductor) image sensor and may capture an image of the same imaging range as the vision sensor 11.

Moreover, the feature amount calculating unit 23 calculates a mean velocity, a body length, a body width, a frequency of an angular change of the cluster 41, and a frequency as feature amounts and classifies the clusters 41 on the basis of the feature amounts. However, the feature amount calculating unit 23 calculates one or more of a mean velocity, a body length, a body width, a frequency of an angular change of the cluster 41, and a frequency as feature amounts and classifies the clusters 41 on the basis of the calculated feature amounts. In other words, the feature amount calculating unit 23 may calculate one or more of the movement-related feature amount, the frequency-related feature amount, the event-related feature amount, and the shape-related feature amount.

### <10. Summary of Embodiments

The measurement device 1 according to the embodiment includes the vision sensor 11 that has a plurality of pixels, each outputting an event signal in an asynchronous manner according to a luminance change of incident light, the cluster forming unit 21 that forms, as the cluster 41, a point group of events generated by identical target particles on the basis of an event signal input in a predetermined period, the feature amount calculating unit 23 that calculates the feature amount of the cluster, and the cluster classifying unit 24 that classifies the cluster on the basis of the feature amount calculated by the feature amount calculating unit.

Thus, the measurement device 1 can classifies the cluster 41 on the basis of the event signal input from the vision sensor 11.

This allows the measurement device 1 to accurately classify the cluster.

The cluster tracking unit 22 that tracks the cluster 41 is further provided. The feature amount calculating unit 23 calculates the feature amount of the cluster tracked by the cluster tracking unit.

Thus, the measurement device 1 can calculate feature amounts (e.g., a mean velocity or a frequency) based on the movement of the cluster 41.

Moreover, the cluster tracking unit 22 aggregates the plurality of clusters 41 based on identical target particles into one cluster on the basis of a feature amount.

Thus, the measurement device 1 can reduce erroneous detection of the plurality of clusters 41 based on identical target particles as the clusters 41 based on different target particles.

When the clusters 41 are aggregated, the feature amount calculating unit 23 recalculates the feature amount on the basis of information about the plurality of aggregated clusters.

Thus, the measurement device 1 calculates a feature amount on the basis of information (event signal) about the clusters 41 before and after aggregation, so that the clusters 41 can be observed for an extended period and the feature amount can be accurately calculated.

The feature amount calculating unit 23 calculates one or more of the feature amounts classified as a movement-related feature amount, a frequency-related feature amount, an event-related feature amount, and a shape-related feature amount of the cluster 41.

This allows the measurement device 1 to accurately classify the cluster 41 by properly selecting an effective feature amount in the classification of target particles.

Moreover, the feature amount calculating unit 23 calculates, as a feature amount, one or more of a mean velocity, a body length, a body width, a frequency of an angular change of the cluster 41, and a change frequency of the number of events constituting the cluster.

This allows the measurement device 1 to classify the cluster 41 on the basis of an effective feature amount in the classification of target particles.

Moreover, the feature amount calculating unit 23 calculates, as a feature amount, all of a mean velocity, a body length, a body width, a frequency of an angular change of the cluster 41, and a change frequency of the number of events constituting the cluster.

This allows the measurement device 1 to more accurately classify the cluster 41 on the basis of a combination of a plurality of effective feature amounts in the classification of target particles.

The power supply control unit 102 that controls the supply of power from the power supply 101 is further provided. The power supply control unit 102 switches the power saving mode for stopping or reducing the supply of power to the units of the measurement device 100 and the normal processing mode for supplying power to the units of the measurement device 100.

Thus, the measurement device 100 can reduce power consumption when measurement is not conducted or in a state where measurement is not necessary, thereby extending the measurement period.

Furthermore, the power supply control unit 102 switches the modes on the basis of the number of clusters formed by the cluster forming unit 21 or a feature amount calculated by the feature amount calculating unit 23.

Thus, the measurement device 100 can conduct measurement in an environment that enables optimum measurement, for example, in the presence of a large number of target particles. In other cases, power consumption can be reduced by the power saving mode.

The measurement device further includes the illumination unit 3 that irradiates the imaging range of the vision sensor 11 with light, and the illumination control unit 201 that controls the amount of light emitted from the illumination unit 3. The illumination control unit 201 changes the amount of light when the changing condition is established.

Thus, the measurement device 200 can optimize the number of events detected by the vision sensor 11, thereby improving the accuracy of measurement with low power consumption.

The changing condition is at least one of the number of event signals equal to or larger than the first predetermined number or equal to or smaller than the second predetermined number, the event signals being output from the vision sensor 11, the ratio of failures in tracking of the cluster, the ratio being equal to or larger than the first predetermined value or equal to or smaller than the second predetermined value, and the cluster having a predetermined size or larger with a sufficient momentum while an extremely large number of events is likely to be provided.

Thus, if the number of events is extremely large or small, the measurement device 200 can optimize the number of events by changing the amount of light emitted from the illumination unit 3.

The control unit 14 that controls the event threshold of the vision sensor 11 is provided. The control unit 14 changes the event threshold when the changing condition is established.

Thus, the measurement device 200 can optimize the number of events detected by the vision sensor 11, thereby improving the accuracy of measurement with low power consumption.

The measurement device further includes the illumination unit 301 including the plurality of light sources 301a that can be separately turned on or off, and the illumination control unit 201 that controls the on/off of the light sources 301a. The illumination control unit 201 changes the on/off of the light sources when a predetermined condition is established.

Thus, the measurement device 300 change the position of light emitted from the illumination unit, enabling measurement focusing on, for example, organisms exhibiting phototaxis.

The illumination control unit 201 changes the on/off of the light sources 301a, a wavelength, and the amount of light when the predetermined condition is established.

Thus, the measurement device 300 can improve the accuracy of measurement on target particles by emitting light corresponding to specific target particles (organisms exhibiting phototaxis).

The measurement device further includes the particle determination unit 411 that classifies target particles classified by the cluster classifying unit 24, as active particles or passive particles.

Thus, the measurement device 400 can calculate the ratio of organisms or non-living objects and the ratio of particles built up on the sea bottom.

The particle count unit 412 that counts the number of active particles and passive particles is further provided.

Thus, the measurement device 400 can calculate the ratio of organisms or non-living objects and the ratio of particles built up on the sea bottom.

The sensor (the gravity sensor 16, the GNSS 402, the bathometer 403, the pH meter 404, and the real-time clock 405) that measures external environment is further provided and stores the number of active particles and passive particles in relation to information about the external environment measured by the sensor.

Thus, the measurement device can calculate, for example, the ratio of active particles and passive particles that are affected by an external environment, biodiversity, and the degree of environmental pollution.

The measurement device further includes the carbon content estimation unit 511 that estimates a carbon content under water on the basis of the number of passive particles and the feature amounts.

Hence, the measurement device 500 can estimate a carbon content at sea, contributing to a reduction of greenhouse gas.

A measurement method includes: forming a point group of events generated by identical target particles on the basis of an event signal input in a predetermined period by the vision sensor that has a plurality of pixels, each outputting an event signal in an asynchronous manner according to a luminance change of incident light; calculating a feature amount of the cluster; and classifying the cluster on the basis of the feature amount.

A program causes the measurement device to form a point group of events generated by identical target particles on the basis of an event signal input in a predetermined period by the vision sensor that has a plurality of pixels, each outputting an event signal in an asynchronous manner according to a luminance change of incident light; calculate a feature amount of the cluster; and classify the cluster on the basis of the feature amount.

The program can be recorded in advance in an HDD serving as a recording medium embedded in a device such as a computer device or a ROM or the like in a microcomputer that includes a CPU.

Alternatively, the program can be stored (recorded) temporarily or perpetually on a removable recording medium such as a flexible disc, a compact disc read-only memory (CD-ROM), a magneto optical (MO) disc, a digital versatile disc (DVD), a Blu-ray Disc (registered trademark), a magnetic disk, a semiconductor memory, or a memory card. The removable recording medium can be provided as so-called package software.

The program can be installed from the removable recording medium to a personal computer or the like and can also be downloaded from a download site via a network such as a local area network (LAN) or the Internet.

Note that the advantageous effects described in the present specification are merely exemplary and are not limited, and other advantageous effects may be obtained.

### < 11. Present Technique>

The present technique can also adopt the following configurations.
(1) A measurement device including: a vision sensor that has a plurality of pixels, each outputting an event signal in an asynchronous manner according to a luminance change of an incident light;
   a cluster forming unit that forms, as a cluster, a point group of events generated by identical target particles on the basis of the event signal input in a predetermined period;
   a feature amount calculating unit that calculates a feature amount of the cluster, and
   a cluster classifying unit that classifies the cluster on the basis of the feature amount calculated by the feature amount calculating unit.
(2) The measurement device according to (1), further including a cluster tracking unit that tracks the cluster,
   wherein the feature amount calculating unit calculates the feature amount of the cluster tracked by the cluster tracking unit.
(3) The measurement device according to (2), wherein the cluster tracking unit aggregates a plurality of the clusters based on identical target particles into the single cluster on the basis of the feature amount.
(4) The measurement device according to (3), wherein when the clusters are aggregated, the feature amount calculating unit recalculates the feature amount on the basis of information about a plurality of the aggregated clusters.
(5) The measurement device according to any one of (1) to (4), wherein the feature amount calculating unit calculates one or more of the feature amounts classified as a movement-related feature amount, a frequency-related feature amount, an event-related feature amount, and a shape-related feature amount of the cluster.
(6) The measurement device according to any one of (1) to (5), wherein the feature amount calculating unit calculates, as the feature amount, one or more of a mean velocity, a body length, a body width, a frequency of an angular change of the cluster, and a change frequency of the number of events constituting the cluster.
(7) The measurement device according to (6), wherein the feature amount calculating unit calculates, as the feature amount, all of the mean velocity, the body length, the body width, the frequency of the angular change of the cluster, and the change frequency of the number of events constituting the cluster.
(8) The measurement device according to any one of (1) to (7), further including a power supply control unit that controls supply of power from a power supply, wherein
   the power supply control unit switches a power saving mode for stopping or reducing supply of power to the units of the measurement device and a normal processing mode for supplying power to the units of the measurement device.
(9) The measurement device according to (8), wherein the power supply control unit switches the modes on the basis of a number of the clusters formed by the cluster forming unit or the feature amount calculated by the feature amount calculating unit.
(10) The measurement device according to any one of (1) to (9), further including: an illumination unit that irradiates an imaging range of the vision sensor with light; and
   an illumination control unit that controls the amount of light emitted from the illumination unit,
   wherein
   the illumination control unit changes the amount of light when a changing condition is established.
(11) The measurement device according to (10), wherein
   the changing condition is at least one of a number of the event signals equal to or larger than a first predetermined number or equal to or smaller than a second predetermined number, the event signals being output from the vision sensor, the ratio of failures in tracking of the cluster, the ratio being equal to or larger than a first predetermined value or equal to or smaller than a second predetermined value, and the cluster having a predetermined size or larger with a sufficient momentum while an extremely large number of the events is likely to be provided.
(12) The measurement device according to any one of (1) to (11), further including a control unit that controls an event threshold of the vision sensor,
   wherein
   the control unit changes the event threshold when a changing condition is established.
(13) The measurement device according to any one of (1) to (12), further including an illumination unit including a plurality of light sources capable of being separately turned on or off; and
   an illumination control unit that controls the on or off of the light sources, wherein
   on or off of the light sources is changed when a predetermined condition is established.
(14) The measurement device according to (13), wherein
   the illumination control unit changes the on or off of the light sources, a wavelength, and the amount of light when the predetermined condition is established.
(15) The measurement device according to any one of (1) to (14), further including a particle determination unit that classifies target particles classified by the cluster classifying unit, as active particles or passive particles.
(16) The measurement device according to (15), further including a particle count unit that counts a number of the active particles and passive particles.
(17) The measurement device according to (16), further including a sensor that measures an external environment,
   wherein the sensor stores the number of active particles and passive particles in relation to information about the external environment measured by the sensor.
(18) The measurement device according to (16) or (17), further including a carbon content estimation unit that estimates a carbon content under water on the basis of the number of passive particles and the feature amount.
(19) A measurement method causing a measurement device to perform the steps of: forming, as a cluster, a point group of events generated by identical target particles on a basis of an event signal input in a predetermined period by a vision sensor that has a plurality of pixels, each outputting an event signal in an asynchronous manner according to a luminance change of incident light; calculating a feature amount of the cluster; and
   classifying the cluster on the basis of the feature amount.
(20) A program that forms, as a cluster, a point group of events generated by identical target particles on a basis of an event signal input in a predetermined period by a vision sensor that has a plurality of pixels, each outputting an event signal in an asynchronous manner according to a luminance change of incident light; calculate a feature amount of the cluster; and
   classify the cluster on the basis of the feature amount.

### [Reference Signs List]

1 Measurement device
3 Illumination unit
11 Vision sensor
13 Signal processing unit
14 Control unit
21 Cluster forming unit
22 Cluster tracking unit
23 Feature amount calculating unit
24 Cluster classifying unit

## Claims

1. A measurement device comprising: a vision sensor that has a plurality of pixels, each outputting an event signal in an asynchronous manner according to a luminance change of an incident light;
a cluster forming unit that forms, as a cluster, a point group of events generated by identical target particles on a basis of the event signal input in a predetermined period;
a feature amount calculating unit that calculates a feature amount of the cluster, and
a cluster classifying unit that classifies the cluster on a basis of the feature amount calculated by the feature amount calculating unit.

2. The measurement device according to claim 1, further comprising a cluster tracking unit that tracks the cluster,
wherein the feature amount calculating unit calculates the feature amount of the cluster tracked by the cluster tracking unit.

3. The measurement device according to claim 2, wherein the cluster tracking unit aggregates a plurality of the clusters based on identical target particles into the single cluster on a basis of the feature amount.

4. The measurement device according to claim 3, wherein when the clusters are aggregated, the feature amount calculating unit recalculates the feature amount on a basis of information about a plurality of the aggregated clusters.

5. The measurement device according to claim 1, wherein the feature amount calculating unit calculates one or more of the feature amounts classified as a movement-related feature amount, a frequency-related feature amount, an event-related feature amount, and a shape-related feature amount of the cluster.

6. The measurement device according to claim 1, wherein the feature amount calculating unit calculates, as the feature amount, one or more of a mean velocity, a body length, a body width, a frequency of an angular change of the cluster, and a change frequency of the number of events constituting the cluster.

7. The measurement device according to claim 6, wherein the feature amount calculating unit calculates, as the feature amount, all of the mean velocity, the body length, the body width, the frequency of the angular change of the cluster, and the change frequency of the number of events constituting the cluster.

8. The measurement device according to claim 1, further comprising a power supply control unit that controls supply of power from a power supply,
wherein
the power supply control unit switches a power saving mode for stopping or reducing supply of power to units of the measurement device and a normal processing mode for supplying power to the units of the measurement device.

9. The measurement device according to claim 8, wherein the power supply control unit switches the modes on a basis of a number of the clusters formed by the cluster forming unit or the feature amount calculated by the feature amount calculating unit.

10. The measurement device according to claim 1, further comprising: an illumination unit that irradiates an imaging range of the vision sensor with light; and
an illumination control unit that controls an amount of light emitted from the illumination unit,
wherein
the illumination control unit changes the amount of light when a changing condition is established.

11. The measurement device according to claim 10, wherein
the changing condition is at least one of a number of the event signals equal to or larger than a first predetermined number or equal to or smaller than a second predetermined number, the event signals being output from the vision sensor, a ratio of failures in tracking of the cluster, the ratio being equal to or larger than a first predetermined value or equal to or smaller than a second predetermined value, and the cluster having a predetermined size or larger with a sufficient momentum while an extremely large number of the events is likely to be provided.

12. The measurement device according to claim 1, further comprising a control unit that controls an event threshold of the vision sensor,
wherein
the control unit changes the event threshold when a changing condition is established.

13. The measurement device according to claim 1, further comprising an illumination unit including a plurality of light sources capable of being separately turned on or off, and an illumination control unit that controls on or off of the light sources,
wherein
on or off of the light sources is changed when a predetermined condition is established.

14. The measurement device according to claim 13, wherein
the illumination control unit changes on or off of the light sources, a wavelength, and an amount of light when the predetermined condition is established.

15. The measurement device according to claim 1, further comprising a particle determination unit that classifies target particles classified by the cluster classifying unit, as active particles or passive particles.

16. The measurement device according to claim 15, further comprising a particle count unit that counts a number of the active particles and the passive particles.

17. The measurement device according to claim 16, further comprising a sensor that measures an external environment,
wherein the sensor stores the number of the active particles and the passive particles in relation to information about the external environment measured by the sensor.

18. The measurement device according to claim 16, further comprising a carbon content estimation unit that estimates a carbon content under water on a basis of the number of the passive particles and the feature amount.

19. A measurement method causing a measurement device to perform the steps of:
forming, as a cluster, a point group of events generated by identical target particles on the basis of an event signal input in a predetermined period by a vision sensor that has a plurality of pixels, each outputting an event signal in an asynchronous manner according to a luminance change of incident light;
calculating a feature amount of the cluster; and
classifying the cluster on a basis of the feature amount.

20. A program that forms, as a cluster, a point group of events generated by identical target particles on a basis of an event signal input in a predetermined period by a vision sensor that has a plurality of pixels, each outputting an event signal in an asynchronous manner according to a luminance change of incident light;
calculate a feature amount of the cluster; and
classify the cluster on a basis of the feature amount.
